(19) Europäisches Patentamt
European Patent Office
Office européen des brevets

(11) **EP 4 219 703 A2**

(12) **EUROPEAN PATENT APPLICATION**

(43) Date of publication:
**02.08.2023 Bulletin 2023/31**

(21) Application number: **22214978.3**

(22) Date of filing: **05.10.2018**

(51) International Patent Classification (IPC):
***C12N 9/36*** *(2006.01)*     ***A61K 38/48*** *(2006.01)*
***C12N 9/52*** *(2006.01)*

(52) Cooperative Patent Classification (CPC):
**C12N 9/2462; A61K 38/162; C12N 9/52**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2017 EP 17195324**

(62) Document number(s) of the earlier application(s) in accordance with Art. 76 EPC:
**18780136.0 / 3 692 149**

(71) Applicant: **Micreos Human Health B.V.**
**2585 EV Den Haag (NL)**

(72) Inventor: **OFFERHAUS, Mark, Leonard**
**2566 SN Den Haag (NL)**

(74) Representative: **Nederlandsch Octrooibureau**
**P.O. Box 29720**
**2502 LS The Hague (NL)**

Remarks:
• The complete document including Reference Table(s) and the Sequence Listing(s) can be downloaded from the EPO website
• This application was filed on 20-12-2022 as a divisional application to the application mentioned under INID code 62.

(54) **TREATMENT OF A CONDITION ASSOCIATED WITH INFECTION WITH AN ONCOGENIC BACTERIUM**

(57) The present invention relates to the field of medicine, specifically to the field of treatment of a malignant condition associated with infection with a bacterium that aggravates and/or induces proliferation of the malignant conditions.

EP 4 219 703 A2

**Description**

**Field of the invention**

The present invention relates to the field of medicine, specifically to the field of treatment of a malignant condition associated with infection with a bacterium that aggravates and/or induces proliferation of the malignant conditions.

**Background of the invention**

Oncogenic bacteria can be a major problem in cancer patients. In several types of cancer, bacteria play a role in pathogenesis and/or oncogenesis. *Helicobacter pylori* infection is e.g. closely related with chronic gastritis, gastric duodenal ulcer, and mucosa associated diseases such as lymphoma and gastric cancer (Nervi G, et al. 2006, Does Helicobacter pylori infection eradication modify peptic ulcer prevalence? A 10 years' endoscopical survey. World J Gastroenterol 12: 2398-2401; Kamangar et al. 2007, Helicobacter pylori and oesophageal and gastric cancers in a prospective study in China. Br J Cancer 96: 172-176). It has been classified as class I carcinogen in 1994 by the World Health Organization and the International Agency for Research on Cancer consensus group (Uemura N, et al. 2001, Helicobacter pylori infection and the development of gastric cancer. N Engl J Med 345: 784-789.).

Recently, Staphylococcal enterotoxin A (SEA) has been demonstrated to stimulates Signal transducer and activator of transcription 3 (STAT3) activation and expression of STAT3 driven effector molecules, such as IL-17 and IL-10, in cutaneous T-cell lymphoma (CTCL) (Willerslev-Olsen, et al. 2016, Staphylococcal enterotoxin A (SEA) stimulates STAT3 activation and IL-17 expression in cutaneous T-cell lymphoma. Blood, 10 March 2016, vol. 127-10; Krejsgaard T, et al., Staphylococcal enterotoxins stimulate lymphoma-associated immune dysregulation; Blood. 2014 Jul 31;124(5):761-70). Hence, SEA promotes activation of an established oncogenic pathway previously implicated in carcinogenesis and *Staphylococcus aureus* can thus be regarded as an oncogenic bacterium according to the definitions herein.

Treatment of these bacterial infections is suggested by use of antibiotics, but antibiotics have the disadvantage that there is factual chance that the target bacteria become resistant (e.g. MRSA). In addition, antibiotics are not very specific and will disturb the balance of commensal benign bacteria; said balance is already fragile and compromised in cancer patients. Moreover, there is no evidence to date that treating CTCL patients with antibiotics has an influence on carcinogenesis. Accordingly, there is a need to a novel method of treatment of cancer patients where oncogenic bacteria are associated with pathogenesis, progression and/or oncogenesis of the cancer.

**Brief description of the figures**

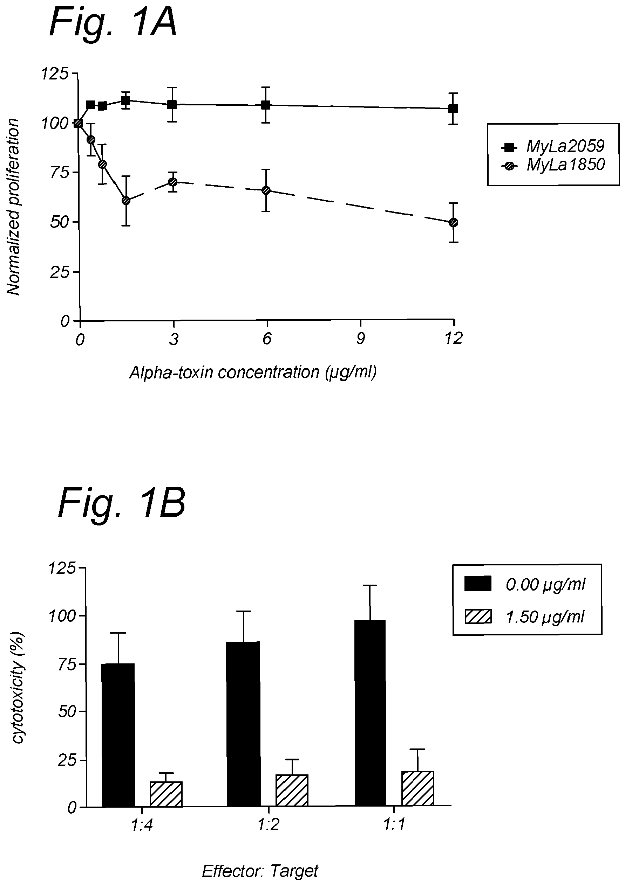

> **Figure 1A:** Proliferation of a malignant and a non-malignant (Mycosis Fungoides) patient derived CTCL cell line with and without alpha-toxin from *Staphylococcus aureus.*
> **Figure 1B:** Cytotoxicity assay of MART-1 specific CD8 T cells directed against peptide pulsed Mac1 cells with and without alpha-toxin from *Staphylococcus aureus.*

**Description of the invention**

It has been established by the inventors that, surprisingly, an endolysin can effectively be used in the treatment of cancer. Without being bound by theory, it is believed that the specificity of the bacteriophage endolysin in killing target bacteria together with its effectiveness in killing target bacteria provides the unexpected superior beneficial effect in the treatment of cancer. Accordingly, the invention provides for an endolysin specific for an oncogenic bacterium for use in the prevention, delay or treatment of cancer in a subject in need thereof.

In all embodiments of the invention, the terms lysin, endolysin and autolysin are used interchangeably. The endolysin specific for an oncogenic bacterium is herein referred as the endolysin according to the invention. The oncogenic bacterium is herein referred to as the oncogenic bacterium according to the invention. In the context of the invention, an oncogenic bacterium is an oncogenic bacterium according to general knowledge in the field, but also a bacterium that in a malignancy associated with infection with said bacterium, aggravates and/or induces proliferation of the malignant condition.

In all embodiments of the invention, the oncogenic bacterium may be any oncogenic bacterium. It may be a bacterium selected from the group consisting of Staphylococcus, Streptococcus (such as *S. pyogenes, S. agalactiae* and *S.pneumonia)*, Actinomyces, Nocardia, Bacillus (such as *B. anthracis)*, Coxiella (such as *Coxiella burnetii)*, Rickettsia, Mycobacteria (such as *M. tuberculosis* and *M. leprae)*, Legionella (such as *L. pneumophila)*, Mycoplasma, Salmonella (such as *S. typhimurium*), Shigella (such as *S. dysenteriae*), Yersinia (such as *Y. pestis*), Brucella, Listeria (such as *L. monocytogenesis*), Actinobacillus (such as *A. actinomycetemcomitans*), Gardnerella (such as *G. vaginalis*), Chlamydia (such as *C. trachomatis*), and Chlamidophila; a more preferred bacterium is a species of Staphylococcus; a preferred species of Staphylococcus is *S. aureus*. A preferred *S. aureus* is an antibiotic resistant *Staphylococcus aureus*, such as a methicillin resistant *Staphylococcus aureus* (MRSA).

Preferably, the endolysin according to the invention is a Staphylococcus-specific endolysin, meaning that it will lyse Staphylococcus efficiently but does not substantially lyse other bacteria than Staphylococcus. Most native *Staphylococcus* bacteriophage endolysins exhibiting peptidoglycan hydrolase activity consist of a C-terminal cell wall-binding domain (CBD), a central N-acetylmuramoyl-L-Alanine amidase domain, and an N-terminal Alanyl-glycyl endopeptidase domain with cysteine, histidine-dependent amidohydrolases/peptidase (CHAP) homology, or in case of Ply2638, of an N-terminal glycyl-glycine endopeptidase domain with Peptidase_M23 homology, the latter three domains exhibiting peptidoglycan hydrolase activity each with distinct target bond specificity and generally named as enzymatically active domains. The Ply2638 endolysin is set forward in SEQ ID NO: 1 and SEQ ID NO: 2 (see Table 1); several endolysin domains are set forward in SEQ ID NO: 3 to SEQ ID NO: 18 (see Table 1), these domains are preferred domains according to the invention.

A more preferred endolysin according to the invention is a recombinant endolysin, more preferably a recombinant Staphylococcus-specific endolysin, even more preferably a recombinant Staphylococcus-specific chimeric endolysin comprising one or more heterologous domains. In general, endolysins are comprised of different subunits (domains); e.g. a cell wall-binding domain (CBD) and one or more enzymatic domains having peptidoglycan activity, such as an amidase domain, an M23 peptidase domain and a CHAP (cysteine, histidine-dependent amidohydrolases/peptidases) domain. An example of a Staphylococcus-specific chimeric endolysin comprising one or more heterologous domains is an endolysin comprising an Amidase domain of bacteriophage Ply2638, an M23 peptidase domain of lysostaphin (*S. simulans*) and a cell wall-binding domain of bacteriophage Ply2638. Such Staphylococcus-specific chimeric endolysin is a preferred endolysin according to the invention and is extensively described in WO2012/150858, which is herein incorporated by reference in its entirety. Other preferred endolysins according to the invention are extensively described in WO2013/169104, which is herein incorporated by reference in its entirety. Other preferred endolysins according to the invention are extensively described in WO2017/046021, which is herein incorporated by reference in its entirety.

The present invention may conveniently be combined with the embodiments and endolysins of WO2016/142445, which is herein incorporated by reference in its entirety. In WO2016/142445, intracellular *Staphylococcus aureus* infections are combatted using an agent the increases the intracellular pH combined with an endolysin that is capable of entry into the cell. Such endolysins that are capable of entering the cell are preferred endolysins according to the invention.

A further preferred endolysin according to the invention is one selected from the group consisting of the endolysins depicted as SEQ ID NO: 19 to SEQ ID NO: 75 in Table 1.

Preferably, an endolysin domain according to the invention has at least 80%, 81%, 82%, 83%, 84%, 85%, 86%, 87%, 88%, 89%, 90%, 91%, 92%, 93%, 94%, 95%, 96%, 97%, 98%, 99% or more preferably 100% sequence identity with a domain depicted in WO2012/150858, WO2013/169104, WO2016/142445 or in any of SEQ ID NO: 3 to SEQ ID NO: 18 (see Table 1).

Preferably, an endolysin according to the invention has at least 80%, 81%, 82%, 83%, 84%, 85%, 86%, 87%, 88%, 89%, 90%, 91%, 92%, 93%, 94%, 95%, 96%, 97%, 98%, 99% or more preferably 100% sequence identity with an endolysin depicted in WO2012/150858, WO2013/169104, WO2016/142445 or in any of SEQ ID NO: 1, 2 and SEQ ID NO: 19 to SEQ ID NO: 75 (see Table 1).

The person skilled in the art will comprehend that mixes of different endolysins may be used in the present invention, e.g. a mix comprising two, three or four endolysins according to the invention. In the embodiments of the invention, the subject is a vertebrate, preferably a mammal, more preferably a human.

The type of cancer to be treated by be any type of cancer where oncogenic bacteria are associated with the pathogenesis and oncogenesis of the cancer. A preferred cancer is a mucosa associated cancer such as (skin)lymphoma and gastric cancer. A more preferred cancer is cutaneous T-cell lymphoma (CTCL).

The person skilled in the art will comprehend that treatment with the endolysin according to the invention may conveniently be combined with other drugs, such as other anti-cancer medicaments. The compounds be present in individual compositions or may be present in a single composition. Accordingly, an endolysin according to the invention may be present in a composition further comprising an excipient, preferably a pharmaceutically acceptable excipient. In said composition according to the invention or pharmaceutical composition according to the invention, a further anti-cancer medicament may be present, such as a chemotherapeutic agent and a monoclonal antibody.

The medical treatment according to the invention includes an endolysin specific for an oncogenic bacterium for the manufacture of a medicament for the prevention, delay or treatment of cancer in a subject in need thereof as well as a method for the prevention, delay or treatment of cancer in a subject in need thereof, comprising administration of an endolysin specific for an oncogenic bacterium to the subject. The embodiments are preferably those as defined here above.

**Table 1: SEQ ID NO: overview**

| SEQ ID NO | Name construct | organism |
|---|---|---|
| 1 | Ply2638 endolysin CDS | Bacteriophage 2638A |
| 2 | Ply2638 endolysin PRT | Bacteriophage 2638A |
| 3 | CWT-LST CDS | *S.* simulans |
| 4 | CWT-LST PRT | *S.* simulans |
| 5 | CBD2638 CDS | Bacteriophage 2638A |
| 6 | CBD2638 PRT | Bacteriophage 2638A |
| 7 | CWT-NM3 CDS | *S. aureus* phage phiNM3 |
| 8 | CWT-NM3 PRT | *S. aureus* phage phiNM3 |
| 9 | CHAPK CDS | *S.* phage K |
| 10 | CHAPK PRT | *S.* phage K |
| 11 | CHAP-ΦTwort CDS | S. phage Twort |
| 12 | CHAP-ΦTwort PRT | S. phage Twort |
| 13 | M23-2638 CDS | Bacteriophage 2638A |
| 14 | M23-2638 PRT | Bacteriophage 2638A |
| 15 | M23-LST CDS | *S. simulans* |
| 16 | M23-LST PRT | *S. simulans* |
| 17 | Ami2638 CDS | Bacteriophage 2638A |
| 18 | Ami2638 PRT | Bacteriophage 2638A |
| 19 | CHAPK_CHAPK_CWT-LST CDS | artificial construct |
| 20 | CHAPK_CHAPK_CWT-LST PRT | artificial construct |
| 21 | M23-LST_M23-LST_CWT-LST CDS | artificial construct |
| 22 | M23-LST_M23-LST_CWT-LST PRT | artificial construct |
| 23 | Ami2638_ami2638_CWT-LST CDS | artificial construct |
| 24 | Ami2638_ami2638_CWT-LST PRT | artificial construct |
| 25 | HXaAmi2638_CBD2638 CDS | artificial construct |
| 26 | HXaAmi2638_CBD2638 PRT | artificial construct |
| 27 | HXaAmi2638_CWT-LST CDS | artificial construct |
| 28 | HXaAmi2638_CWT-LST PRT | artificial construct |
| 29 | HXaAmi2638_CWT-NM3 CDS | artificial construct |
| 30 | HXaAmi2638_CWT-NM3 PRT | artificial construct |
| 31 | HXaCHAPK_CBD2638 CDS | artificial construct |
| 32 | HXaCHAPK_CBD2638 PRT | artificial construct |
| 33 | HXaCHAPK_CWT-LST CDS | artificial construct |
| 34 | HXaCHAPK_CWT-LST PRT | artificial construct |
| 35 | HXaCHAPK_CWT-NM3 CDS | artificial construct |
| 36 | HXaCHAPK_CWT-NM3 PRT | artificial construct |
| 37 | HXaCHAPTw_CBD2638 CDS | artificial construct |
| 38 | HXaCHAPTw_CBD2638 PRT | artificial construct |
| 39 | HXaCHAPTw_CWT-LST CDS | artificial construct |
| 40 | HXaCHAPTw_CWT-LST PRT | artificial construct |
| 41 | HXaCHAPTw_CWT-NM3 CDS | artificial construct |
| 42 | HXaCHAPTw_CWT-NM3 PRT | artificial construct |
| 43 | HXaM23-LST_CBD2638 CDS | artificial construct |
| 44 | HXaM23-LST_CBD2638 PRT | artificial construct |
| 45 | HXaM23-LST_CWT-LST CDS | artificial construct |
| 46 | HXaM23-LST_CWT-LST PRT | artificial construct |
| 47 | HXaM23-LST_CWT-NM3 CDS | artificial construct |
| 48 | HXaM23-LST_CWT-NM3 PRT | artificial construct |
| 49 | HXaAmi2638_Ami2638_CBD2638 CDS | artificial construct |

(continued)

| SEQ ID NO | Name construct | organism |
|---|---|---|
| 50 | HXaAmi2638_Ami2638_CBD2638 PRT | artificial construct |
| 51 | HXaAmi2638_Ami2638_CWT-LST CDS | artificial construct |
| 52 | HXaAmi2638_Ami2638_CWT-LST PRT | artificial construct |
| 53 | HXaAmi2638_Ami2638_CWT-NM3 CDS | artificial construct |
| 54 | HXaAmi2638_Ami2638_CWT-NM3 PRT | artificial construct |
| 55 | HXaCHAPK_CHAPK_CBD2638 CDS | artificial construct |
| 56 | HXaCHAPK_CHAPK_CBD2638 PRT | artificial construct |
| 57 | HXaCHAPK_CHAPK_CWT-LST CDS | artificial construct |
| 58 | HXaCHAPK_CHAPK_CWT-LST PRT | artificial construct |
| 59 | HXaCHAPK_CHAPK_CWT-NM3 CDS | artificial construct |
| 60 | HXaCHAPK_CHAPK_CWT-NM3 PRT | artificial construct |
| 61 | HXaCHAPTw_CHAPTw_CBD2638 CDS | artificial construct |
| 62 | HXaCHAPTw_CHAPTw_CBD2638 PRT | artificial construct |
| 63 | HXaCHAPTw_CHAPTw_CWT-LST CDS | artificial construct |
| 64 | HXaCHAPTw_CHAPTw_CWT-LST PRT | artificial construct |
| 65 | HXaCHAPTw_CHAPTw_CWT-NM3 CDS | artificial construct |
| 66 | HXaCHAPTw_CHAPTw_CWT-NM3 PRT | artificial construct |
| 67 | HXaM23-LST_M23-LST_CBD2638 CDS | artificial construct |
| 68 | HXaM23-LST_M23-LST_CBD2638 PRT | artificial construct |
| 69 | HXaM23-LST_M23-LST_CWT-LST CDS | artificial construct |
| 70 | HXaM23-LST_M23-LST_CWT-LST PRT | artificial construct |
| 71 | HXaM23-LST_M23-LST_CWT-NM3 CDS | artificial construct |
| 72 | HXaM23-LST_M23-LST_CWT-NM3 PRT | artificial construct |
| 73 | M23-LST_Ami2638_CBD2638 PRT | artificial construct |
| 74 | M23-LST_Ami2638_CBD2638 PRT* | artificial construct |
| 75 | MART-1 peptide | artificial construct |

Uneven SEQ ID NOs: 1 - 71 represent the coding sequences (CDS) of even SEQ ID NOs: 2 - 72 that represent the respective polypeptide (PRT) sequences.

**Definitions**

"Sequence identity" is herein defined as a relationship between two or more amino acid (peptide, polypeptide, or protein) sequences or two or more nucleic acid (nucleotide, polynucleotide) sequences, as determined by comparing the sequences. In the art, "identity" also means the degree of sequence relatedness between amino acid or nucleotide sequences, as the case may be, as determined by the match between strings of such sequences. "Similarity" between two amino acid sequences is determined by comparing the amino acid sequence and its conserved amino acid substitutes of one peptide or polypeptide to the sequence of a second peptide or polypeptide. In a preferred embodiment, identity or similarity is calculated over the whole SEQ ID NO as identified herein. "Identity" and "similarity" can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps). Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons. Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

A "nucleic acid molecule" or "polynucleotide" (the terms are used interchangeably herein) is represented by a nucleotide sequence. A "polypeptide" is represented by an amino acid sequence. A "nucleic acid construct" is defined as a nucleic acid molecule which is isolated from a naturally occurring gene or which has been modified to contain segments of nucleic acids which are combined or juxtaposed in a manner which would not otherwise exist in nature. A nucleic acid molecule is represented by a nucleotide sequence. Optionally, a nucleotide sequence present in a nucleic acid construct is operably linked to one or more control sequences, which direct the production or expression of said peptide or polypeptide in a cell or in a subject.

"Operably linked" is defined herein as a configuration in which a control sequence is appropriately placed at a position relative to the nucleotide sequence coding for the polypeptide of the invention such that the control sequence directs the production/expression of the peptide or polypeptide of the invention in a cell and/or in a subject. "Operably linked" may also be used for defining a configuration in which a sequence is appropriately placed at a position relative to another sequence coding for a functional domain such that a chimeric polypeptide is encoded in a cell and/or in a subject.

"Expression" is construed as to include any step involved in the production of the peptide or polypeptide including, but not limited to, transcription, post-transcriptional modification, translation, post-translational modification and secretion.

A "control sequence" is defined herein to include all components which are necessary or advantageous for the expression of a polypeptide. At a minimum, the control sequences include a promoter and transcriptional and translational stop signals. Optionally, a promoter represented by a nucleotide sequence present in a nucleic acid construct is operably linked to another nucleotide sequence encoding a peptide or polypeptide as identified herein.

The term "transformation" refers to a permanent or transient genetic change induced in a cell following the incorporation of new DNA (i.e. DNA exogenous to the cell). When the cell is a bacterial cell, as is intended in the present invention, the term usually refers to an extrachromosomal, self-replicating vector which harbors a selectable antibiotic resistance.

An "expression vector" may be any vector which can be conveniently subjected to recombinant DNA procedures and can bring about the expression of a nucleotide sequence encoding a polypeptide of the invention in a cell and/or in a subject. As used herein, the term "promoter" refers to a nucleic acid fragment that functions to control the transcription of one or more genes or nucleic acids, located upstream with respect to the direction of transcription of the transcription initiation site of the gene. It is related to the binding site identified by the presence of a binding site for DNA-dependent RNA polymerase, transcription initiation sites, and any other DNA sequences, including, but not limited to, transcription factor binding sites, repressor and activator protein binding sites, and any other sequences of nucleotides known to one skilled in the art to act directly or indirectly to regulate the amount of transcription from the promoter. Within the context of the invention, a promoter preferably ends at nucleotide -1 of the transcription start site (TSS).

A "polypeptide" as used herein refers to any peptide, oligopeptide, polypeptide, gene product, expression product, or protein. A polypeptide is comprised of consecutive amino acids. The term "polypeptide" encompasses naturally occurring or synthetic molecules.

The sequence information as provided herein should not be so narrowly construed as to require inclusion of erroneously identified bases. The skilled person is capable of identifying such erroneously identified bases and knows how to correct for such errors.

In this document and in its claims, the verb "to comprise" and its conjugations is used in its nonlimiting sense to mean that items following the word are included, but items not specifically mentioned are not excluded. In addition the verb "to consist" may be replaced by "to consist essentially of" meaning that a product or a composition or a nucleic acid molecule or a peptide or polypeptide of a nucleic acid construct or vector or cell as defined herein may comprise additional component(s) than the ones specifically identified; said additional component(s) not altering the unique characteristic of the invention. In addition, reference to an element by the indefinite article "a" or "an" does not exclude the possibility that more than one of the elements is present, unless the context clearly requires that there be one and only one of the elements. The indefinite article "a" or "an" thus usually means "at least one".

All patent and literature references cited in the present specification are hereby incorporated by reference in their entirety.

The following examples are offered for illustrative purposes only, and are not intended to limit the scope of the present invention in any way.

**Example 1**

A subject suffering from cutaneous T-cell lymphoma (CTCL) is treated by topical administration of an endolysin according to the invention specific for *Staphylococcus aureus.* As a result, STAT3 activation and IL-10 and IL-17 expression are efficiently reduced, thereby treating the cancer.

**Example 2** Analysis of the effect of *Staphylococcus aureus* alpha-toxin on malignant T-cells and non-malignant T-cells such as cytotoxic T-cells.

Summary

It was demonstrated for the first time that the *Staphylococcus aureus* alpha-toxin inhibits the proliferation of non-malignant T-cells, such as cytotoxic T-cells, but not of malignant T-cells. In addition, it was demonstrated that *Staphylococcus aureus* alpha-toxin inhibits cytotoxic activity of CTCL-specific cytotoxic CD8 T-cells. This implicates that the killing of cancer cells by cytotoxic T-cells is inhibited by *Staphylococcus aureus* alpha-toxin resulting in escape of tumour cells from killing and that *Staphylococcus aureus* alpha-toxin favours growth and survival of tumour cells. It follows that treatment targeting the alpha-toxin producing *Staphylococcus aureus* of patients with T cell lymphoma effectively treats the CTCL.

Introduction

To investigate the role of *Staphylococcus aureus* alpha-toxin in T-cell lymphoma, it was investigated what the influence of *Staphylococcus aureus* alpha-toxin on proliferation of non-malignant T-cells, such as cytotoxic T-cells, and of malignant T-cells is; specifically whether there is a difference in proliferation in the presence of *Staphylococcus aureus* alpha-toxin.

Proliferation of patient-derived malignant and non-malignant CTCL cell lines was measured in the presence and absence of *Staphylococcus aureus* alpha-toxin by a standard $^3$H-Thymidine incorporation assay. In addition, a CTL assay (LDH, lactate dehydrogenase) release assay was performed using MART-1 specific CD8 T-cells as effector cells and Mac1 cells pulsed with MART-1 peptide (ELAGIGILTV: SEQ ID NO: 75:) as target cells, in the presence and absence of *Staphylococcus aureus* alpha-toxin.

Materials and methods

*Proliferation assay*

Malignant T-cell line MyLa2059 cells (Acc No: RRID:CVCL_5342 (Woetmann et al, 2007)) were plated on 96 well plates at a cell density of 1,250 cells per well, while non-malignant T-cell line MyLa1850 cells (Acc No: RRID:CVCL_M415 (Woetmann et al, 2007)), were plated at a cell density of 2,500 cells per well. Cells were cultured at 37°C in 20 μl medium RPMI-1640 at 5% $CO_2$ as described in Levring et al, 2015, while being exposed to 2-fold increasing concentrations of alpha-toxin (List lab, Cat. No. #120). After 72 hrs of incubation at 37°C, 1 μCi/well Thymidine [Methyl-$^3$H] (PerkinElmer, Cat. No. NET027250UC) was added for an additional 24 hrs. After harvesting the 96 well plate onto a filter plate using a FilterMateHarvester (PerkinElmer), the filter plate was dried overnight; subsequently 20 μl Micro-ScintTM-20 cocktail (PerkinElmer, Cat No. 6013621) to each well on the counting filter. The filter was then analysed with a TopCount®NXTTM Microplate Scintillation and Luminescence Counter (Packard), counting the incorporated thymidine as counts per minutes (cpm), indirectly reflecting cell proliferation. Counts were normalized to the toxin untreated sample within each group.

*CTL assay*

For the cytotoxicity assay, CD8 effector cells (Sorensen et al, 2009) were pre-treated with 1.5 µg/ml alpha-toxin (Sigma, Cat. No. H9395-.5MG) for 2hrs at, while Mac1 target cells (Acc No: RRID:CVCL_H631 (Davis et al, 1992)) were pulsed with 1 µM of MART-1 peptide at 37°C. Cells were plated in phenol free X-VIVO (Lonza, Cat. No. BE02-061Q) media with effector target ratios of 1:4, 1:2 and 1:1. LDH release was quantified from culture supernatants after 4 hrs in co-culture following the manufactures instructions (TaKaRa, Cat. No. MK401). Specific cell lysis (specific cytotoxicity) was calculated after subtracting the background release of the CD8+ T cells using the following formula:

Control:

- Un-pulsed Mac1 cells → exposed to the respective amount of DMSO as the peptide pulsed cells

Background subtraction:

- Background release of CD8 was subtracted prior to applying the equation below
- Formula for calculation of specific cytotoxicity:

$$\% \, Cytotoxicity = \frac{(\text{Experimental release} - \text{spontaneous release})}{(\text{Maximum release} - \text{spontaneous release})} \times 100$$

Experimental release: Co-culture of pulsed Mac1 cells with effector cells
Spontaneous release: Co-culture of DMSO Mac1 cells with effector cells
Maximum release: Mac1 cells treated with Tritox-100 X

The spontaneous release was measured in co-cultures of CD8+ T cells with un-pulsed targets cells and the maximum release in monocultures of effector cells in the presence of Triton X-100 (Sigma-Aldrich, #T8787).

Results and conclusion

*Proliferation assay*

The results of the proliferation assay are depicted in Figure 1A. It is clear that proliferation of malignant MyLa3059cells is not inhibited by *Staphylococcus aureus* alpha-toxin while proliferation of non-malignant MyLa1850 cells is significantly inhibited by *Staphylococcus aureus* alpha-toxin.

*CTL assay*

The results of the CTL assay are depicted in Figure 1B. It is clear that the CD8 effector cells effectively kill the MART-1 peptide pulsed Mac1 target cells. In contrast, when CD8-effector cells have been pre-incubated with *Staphylococcus aureus* alpha-toxin, cytotoxic activity is severely hampered.

Altogether, it has clearly been demonstrated that *Staphylococcus aureus* alpha-toxin inhibits killing of cancer cells by CD8 cytotoxic T-cells. In addition, it has been demonstrated that proliferation of malignant cells is not inhibited by *Staphylococcus aureus* alpha-toxin while proliferation of non-malignant T-cells is inhibited by *Staphylococcus aureus* alpha-toxin, providing a selective advantage to malignant T-cells, resulting in outgrowth of malignant cells at the expense of non-malignant cells. These results provide a clear incentive to treat CTCL, apart from state of the art anti-cancer medication, with an agent, such as an endolysin, that specifically eradicates *Staphylococcus aureus,* while leaving other, commensal, microorganisms intact.

**References:**

Woetmann, A. et al. Nonmalignant T cells stimulate growth of T-cell lymphoma cells in the presence of bacterial toxins. Blood 109, 3325-3332 (2007).

Davis, T. H., Morton, C. C., Miller-Cassman, R., Balk, S. P. & Kadin, M. E. Hodgkin's disease, lymphomatoid papulosis, and cutaneous T-cell lymphoma derived from a common T-cell clone. N. Engl. J. Med. 326, 1115-1122 (1992).

Levring TB1, et al. Human CD4+ T cells require exogenous cystine for glutathione and DNA synthesis. Oncotarget. 2015 Sep 8;6(26):21853-64.).

Sorenson, K et al., The immunodominant HLA-A2-restricted MART-1 epitope is not presented on the surface of many melanoma cell lines Cancer Immunol Immunother (2009) 58:665-675.

**Claims**

1. An endolysin specific for an oncogenic bacterium for use in the prevention, delay or treatment of cancer in a subject in need thereof.

2. An endolysin specific for an oncogenic bacterium for use according to claim 1, wherein the oncogenic bacterium is a *Staphylococcus,* preferably a *Staphylococcus aureus,* more preferably an antibiotic resistant *Staphylococcus aureus,* such as a methicillin resistant *Staphylococcus aureus* (MRSA).

3. An endolysin specific for an oncogenic bacterium for use according to claim 1 or 2, wherein the endolysin is a *Staphylococcus-specific* endolysin.

4. An endolysin specific for an oncogenic bacterium for use according to claim 3, wherein the endolysin is a recombinant *Staphylococcus-specific* endolysin, preferably a recombinant *Staphylococcus-specific* chimeric endolysin comprising one or more heterologous domains.

5. An endolysin specific for an oncogenic bacterium for use according to claim 4, wherein the recombinant *Staphylococcus-specific* endolysin is selected from the group consisting of the endolysins depicted as SEQ ID NO: 19 to SEQ ID NO: 75 in Table 1.

6. An endolysin specific for an oncogenic bacterium for use according to any one of claims 1 - 5, wherein the subject is a human.

7. An endolysin specific for an oncogenic bacterium for use according to any one of claims 1 - 6, wherein the cancer is a mucosa associated cancer such as (skin)lymphoma and gastric cancer.

8. An endolysin specific for an oncogenic bacterium for use according to any one of claims 1 - 7, wherein the cancer is cutaneous T-cell lymphoma (CTCL).

9. An endolysin specific for an oncogenic bacterium for use according to any one of claims 1 - 8, wherein the endolysin is present in a composition further comprising an excipient, preferably a pharmaceutically acceptable excipient.

10. An endolysin specific for an oncogenic bacterium for use according to claim 9, wherein the composition further comprises another anti-cancer medicament.

11. A method for the prevention, delay or treatment of cancer in a subject in need thereof, comprising administration of an endolysin specific for an oncogenic bacterium to the subject.

12. Use of an endolysin specific for an oncogenic bacterium for the manufacture of a medicament for the prevention, delay or treatment of cancer in a subject in need thereof.

## Fig. 1A

## Fig. 1B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012150858 A
- WO 2013169104 A
- WO 2017046021 A
- WO 2016142445 A

### Non-patent literature cited in the description

- **NERVI G et al.** Does Helicobacter pylori infection eradication modify peptic ulcer prevalence? A 10 years' endoscopical survey. *World J Gastroenterol,* 2006, vol. 12, 2398-2401
- **KAMANGAR et al.** Helicobacter pylori and oesophageal and gastric cancers in a prospective study in China. *Br J Cancer,* vol. 96, 172-176
- **UEMURA N et al.** Helicobacter pylori infection and the development of gastric cancer. *N Engl J Med,* 2001, vol. 345, 784-789
- **WILLERSLEV-OLSEN et al.** Staphylococcal enterotoxin A (SEA) stimulates STAT3 activation and IL-17 expression in cutaneous T-cell lymphoma. *Blood,* 10 March 2016, vol. 127-10
- **KREJSGAARD T et al.** Staphylococcal enterotoxins stimulate lymphoma-associated immune dysregulation. *Blood,* 31 July 2014, vol. 124 (5), 761-70
- Computational Molecular Biology. Oxford University Press, 1988
- Biocomputing: Informatics and Genome Projects. Academic Press, 1993
- Computer Analysis of Sequence Data, Part I. Humana Press, 1994
- **VON HEINE, G.** Sequence Analysis in Molecular Biology. Academic Press, 1987
- Sequence Analysis Primer. M Stockton Press, 1991
- **CARILLO, H. ; LIPMAN, D.** *SIAM J. Applied Math.,* 1988, vol. 48, 1073
- **DEVEREUX, J. et al.** *Nucleic Acids Research,* 1984, vol. 12 (1), 387
- **ALTSCHUL, S. F. et al.** *J. Mol. Biol.,* 1990, vol. 215, 403-410
- **ALTSCHUL, S. et al.** BLAST Manual. NCBI NLM NIH
- **ALTSCHUL, S. et al.** *J. Mol. Biol.,* 1990, vol. 215, 403-410
- **NEEDLEMAN ; WUNSCH.** *J. Mol. Biol.,* 1970, vol. 48, 443-453
- **HENTIKOFF ; HENTIKOFF.** *Proc. Natl. Acad. Sci. USA.,* 1992, vol. 89, 10915-10919
- **WOETMANN, A. et al.** Nonmalignant T cells stimulate growth of T-cell lymphoma cells in the presence of bacterial toxins. *Blood,* 2007, vol. 109, 3325-3332
- **DAVIS, T. H. ; MORTON, C. C. ; MILLER-CASSMAN, R. ; BALK, S. P. ; KADIN, M. E.** Hodgkin's disease, lymphomatoid papulosis, and cutaneous T-cell lymphoma derived from a common T-cell clone. *N. Engl. J. Med.,* 1992, vol. 326, 1115-1122
- **LEVRING TB1 et al.** Human CD4+ T cells require exogenous cystine for glutathione and DNA synthesis. *Oncotarget.,* 08 September 2015, vol. 6 (26), 21853-64
- **SORENSON, K et al.** The immunodominant HLA-A2-restricted MART-1 epitope is not presented on the surface of many melanoma cell lines. *Cancer Immunol Immunother,* 2009, vol. 58, 665-675